# EUROPEAN PATENT SPECIFICATION

(11) **EP 1 715 826 B1**
(45) Date of publication and mention of the grant of the patent: **06.03.2013**
(21) Application number: 05722687.0
(22) Date of filing: 31.01.2005
(51) Int. Cl.: A61F 6/22, A61F 6/14

(54) **ENHANCING TISSUE INGROWTH FOR CONTRACEPTION**
VERBESSERUNG DES EINWACHSENS VON GEWEBE ZUR KONTRAZEPTION
AMELIORATION DE LA CROISSANCE DES TISSUS POUR LA CONTRACEPTION

(30) Priority: 02.02.2004 US 541821 P
(43) Date of publication of application: 02.11.2006
(73) Proprietor: Conceptus, Inc., Mountain View, CA 94041 (US)
(72) Inventor: CALLISTER, Jeffrey, P., Deephaven, Minnesota 55391 (US); TREMULIS, William, S., Redwood City, CA 94065 (US)
(74) Representative: Miller, James Lionel Woolverton
(86) International application number: PCT/US2005/003310
(87) International publication number: WO 2005/074845

(56) References cited:
- EP-A- 1 199 049
- WO-A-90/09158
- WO-A1-2005/074845
- US-A- 5 433 217
- US-A1- 2002 020 417
- US-A1- 2002 029 051

## Description

### BACKGROUND OF THE INVENTION

This invention generally relates to the field of occluding devices, delivery systems for such devices for the occlusion of body passageways. The invention is particularly useful for the occluding reproductive lumens such as a female patient's fallopian tubes or a male patient's vas deferens to affect contraception.

Conventional contraceptive strategies generally fall within three categories: physical barriers, drugs and surgery. While each have certain advantages, they also suffer from various drawbacks. Barriers such as condoms and diaphragms are subject to failure due to breakage, displacement and misplacement. Drug strategies, such as the pill and Norplant™, which rely on artificially controlling hormone levels, suffer from known and unknown side-effects from prolonged use. Surgical procedures, such as tubal ligation and vasectomy, are very effective, but involve the costs and attendant risks of surgery, and are frequently not reversible.

Recently, minimally invasive treatments have be proposed which deploy stent-like devices within reproductive lumens for obstructing such lumens as a contraceptive alternative to tubal ligation. However, placing a stent alone or a stent with fibrous material or similar occluding device may not create sufficient or permanent obstruction of the reproductive lumen depending on the nature of the obstructive device. For example, the obstructive device may be too small to provide complete obstruction of the reproductive lumen, or the device may be permeable to cell movement. An occluding device placed in a reproductive lumen, for example, may not securely seal against the luminal walls, or may initially allow egg cells or sperm cells to pass through the device until tissue growth completes the occlusion of the reproductive lumen and thus allow pregnancy to occur. Additionally, the occluding device might create an obstruction sufficient to prevent the passage of an egg but allow sperm cells to pass through or by the occluding device, fertilizing an egg upstream of the obstruction and resulting in an ectopic pregnancy.
The use of an occluding contraceptive or sterilization device, particularly with mesh or fibrous material to promote tissue ingrowth, has been proposed (See for example U. S. Patent No. 6,432, 116). However, with these devices there is an initial period after deployment during which the patient is at risk for cell passage through the device and which can result in pregnancy. US 2002/0020417 describes a known contraceptive transcervical fallopian tube occlusion devices and methods.

### SUMMARY OF THE INVENTION

According to first and second aspects of the present invention, there is provided a contraceptive device for occluding a patients reproductive lumen as claimed in claim 1 and 10 respectively. As used herein, tissue growth includes but is not limited to cell multiplication and/or growth resulting in tissue formation into, onto, or surrounding an occluding device. The tissue growth may be epithelialization, scar formation, or other cell growth or multiplication.

An occluding device having features of the invention generally includes an occluding component which is configured to expand within the body lumen to be occluded and first and second metallic elements which are associated with the occluding component and which are configured to generate electrical activity within a patient's body lumen to enhance the growth of tissue into or onto the occluding component of the occluding device.

In one embodiment having features of this invention, the occluding device has a occluding component and has two metallic components formed of different metallic material and electrically connected to effect galvanic activity when the occluding device contacts body fluid or other conductive fluid to stimulate cellular growth into or on the occluding component of the device. One or both of the metallic elements may be part of the occluding device or separate elements directly or indirectly attached to the occluding device.

A bimetallic spiral intranterine device is described in WO-A- 90/02478.

In another embodiment having features of the invention, the occluding device has a occluding component and has two separate metallic components which are configured to be electrically connected to an electrical power source (e.g. a battery or other low voltage source) to generate the electrical activity sufficient to stimulate cell growth onto or within the occluding component of the device. When the occluding device is deployed within a body lumen and contacts body fluid or other electrically conductive fluid (e.g. saline) therein which acts as an electrolyte, cell growth into or onto the occluding component is stimulated to enhance attachment of the device within the body lumen and occlusion of the lumen.

One particularly useful application for occluding devices embodying features of the invention is directed to occluding a reproductive lumen such as a fallopian tube or a vas deferens for contraceptive purposes.
In some situations, one of the metallic elements or one group of the metallic elements configured for developing electrical activity in the body lumen, may be formed of copper to provide further contraceptive action in addition to the occlusion of the lumen. Additionally, therapeutic or diagnostic agents by be employed with the occluding device for contraceptive or other uses, e.g. antibiotics, chemotherapy. For example, the occluding component can be coated with a polymer having impregnated therein an agent such as a drug, enzyme or protein, for inducing or promoting tissue growth. In yet another refinement, the surface of the occluding component may be plated with or otherwise incorporated with an elutable inflammatory material to produce an inflammatory response in the tissue of the wall defining the body lumen, which further contributes to the obstruction of the lumen. Inflammatory materials include copper or copper alloys. Other inflammatory materials, such as radioactive materials (emitting alpha, beta or gamma particles) may be used alone or in conjunction with other inflammatory materials.

The occluding device embodying features of the invention preferably has an occluding component that at least in part has a first delivery configuration with small transverse dimensions suitable for delivery to the chosen location in a reproductive or other body lumen and a second expanded configuration larger in transverse dimensions than the first configuration to facilitate securing the occluding component of the device within the reproductive or body lumen. The occluding component of the device may be balloon expandable or self-expandable from the first configuration to the second configuration to occlude the body lumen.

The occluding device may be a tubular stent-like structure, such as described in U.S. Patent Applications Serial Nos. 08/770,123, filed on December 18, 1996, 09/112,085, filed on July 8, 1998, 09/468,749, filed on December 21, 1999 and U.S. Provisional Application Serial No. 60/483,587, filed on June 27, 2003. Alternatively, the occluding device may have one or more spider-like constructions shown in co-pending application Serial No. 10/746,131, filed on December 24, 2003. The occluding component will generally be about 1 to about 5 mm, preferably about 2 to about 4 mm in transverse dimension in the expanded configuration and will generally be about 0.5 to about 8 cm, preferably about 1.5 to about 4 cm in length. While the description herein is focused on the use of only one occluding device, two or more occluding devices may be employed in a reproductive or other body lumen.

The occluding device having features of the invention is placed in a contracted configuration to facilitate introduction and advancement of the device within the reproductive lumen or other body lumen, usually within an inner lumen of a delivery sheath. Once in the desired position within a patient's body lumen, the delivery sheath is withdrawn while the occluding device is held in-place at the desired location. The sheath withdrawal exposes the occluding device at the chosen site and the occluding device is expanded either by inflating a balloon within the inner lumen of the occluding component or from self expansion due to the nature and condition of the metallic material of which the occluding component is made. An exposed portion of the occluding device may expand or be expanded before the entire occluding device is discharged from the delivery sheath. Self-expanding occluding components of the device may be formed of superelastic NITINOL with an austenite phase that is stable at body temperature, i.e. the material of the occluding component will not transform from the austenite phase to a martensite phase at body temperature except by the application of stress. The occluding component may also be formed of a heat expandable metallic material such as shape memory NITINOL which has a stable martensite phase at body temperature and which returns to a remembered expanded condition when heated above the martensite-to-austenite transition temperature causing the occluding component to expand within the patient's body lumen.

The metallic elements associated with the occluding component and which generate the electrical activity within the body lumen to stimulate tissue growth may be formed at least in part of suitable conductive metallic materials such as stainless steel, NiTi alloy, platinum, tantalum, copper and gold. Other conductive materials are suitable. Moreover, the metallic elements may be part of the occluding component or be separate elements which are directly or indirectly secured to the occluding component. They may be electrically connected through one or more electrical conductors or even the occluding component itself.

Fibrous strands or bundles thereof, fibrous mesh, porous polymer bodies and the like may be disposed within or on the occluding device to facilitate tissue ingrowth in addition to the electrical stimulation of tissue growth. The fibrous bundle, fibrous mesh or porous polymeric bodies may be disposed in either balloon expandable or self-expanding occluding devices. However, mesh, fibrous or porous polymeric material within the inner lumen of balloon expandable occluding components can make balloon placement within the inner lumen difficult.

To generate the electrical activity, in general, two different metal elements which are electrically connected are immersed in an electrolyte, such as body fluids or saline or both to stimulate tissue growth. The body fluid in which the occlusive device is generally immersed, such as the moist tissue and surface within reproductive lumens may frequently suffice to create the electrical activity of minimal magnitude required for these purposes. Additional conductive fluid such as saline may be provided within the body lumen if needed to facilitate the desired electrical application.

These and other advantages of the invention will become more apparent from the following detailed description of embodiment having features of the invention, taken in conjunction with the accompanying exemplary drawings.

### BRIEF DESCRIPTION OF THE DRAWINGS

Figure 1 is an elevational view of an occluding device embodying features of the invention with an occluding component in a first, contracted configuration.

Figure 2 is a transverse cross sectional view of the device shown in Figure 1, taken along lines 2-2.

Figure 3 is an elevational view of the device shown in Figure 1, in a second, expanded configuration.

Figure 4 is a transverse cross sectional view of the device shown in Figure 3, taken along lines 4--4.

Figure 5 is an elevational view of an alternative occluding device embodying features of the invention having bundles of fibrous strands intermittently spaced in a plurality of sections within the inner lumen of the occluding component.

Figure 6 is an elevational view of another alternative occluding device embodying features of the invention having a fibrous mesh of woven strands disposed at one end of the occluding component.

Figure 7 is a partial end view of the fibrous mesh shown in Figure 6.

Figure 8 is a longitudinal cross sectional view of the occluding device shown in Figure 6, disposed within a body lumen and illustrating tissue growth into and on the occluding component.

Figure 9 is a transverse cross sectional view of the device shown in Figure 8, taken along lines 9--9.

Figure 10 illustrates another alternative occluding device embodying features of the invention which is disposed in an unexpanded configuration within a body lumen and which has a fibrous jacket or sock on an outer surface of the occluding component.

Figure 11 illustrates the device shown in FIG. 10 in an expanded configuration.

Figure 12 is an elevational view of an occluding device embodying features of the invention with a metallic rod within the interior of the occluding component in an unexpanded configuration.

Figure 13 is an elevational view of an occlusive device of Figure 12 in its expanded configuration.

Figure 14 is a transverse cross-sectional view of a modification of the occluding device with the inner rod or wire deployed within a fibrous body in the interior of the occluding component.

Figure 15 illustrates an occluding device embodying features of the invention having a helically shaped occluding component and a helically shaped member of different metallic composition which interfits within the spacing between the turns of the helically shaped occluding component.

Figure 16 illustrates an occluding device embodying features of the invention comprising an occluding component as shown in Figure 15 with a tightly coiled helical member within the inner lumen of the occluding component.

Figure 17 is an isometric view of an alternative occluding device comprising two sheets of metal sheet or foil formed of different metals;

Figure 18 is an isometric view of an alternative occluding device having granules of different metals on its surface;

Figure 19 is a schematic view of a female reproductive anatomy with the occluding device embodying features of the invention configured for deployment within a female patient's fallopian tube.

Figure 20 is an elevational view partially is section of the occluding device shown in Figure 19 disposed within the fallopian tube of a female patient and which has an external electrical source.

Figure 21 is a perspective view of an alternative occluding device having an occluding component with several spider-like members disposed along the supporting shaft.

### DETAILED DESCRIPTION OF EMBODIMENTS OF THE INVENTION

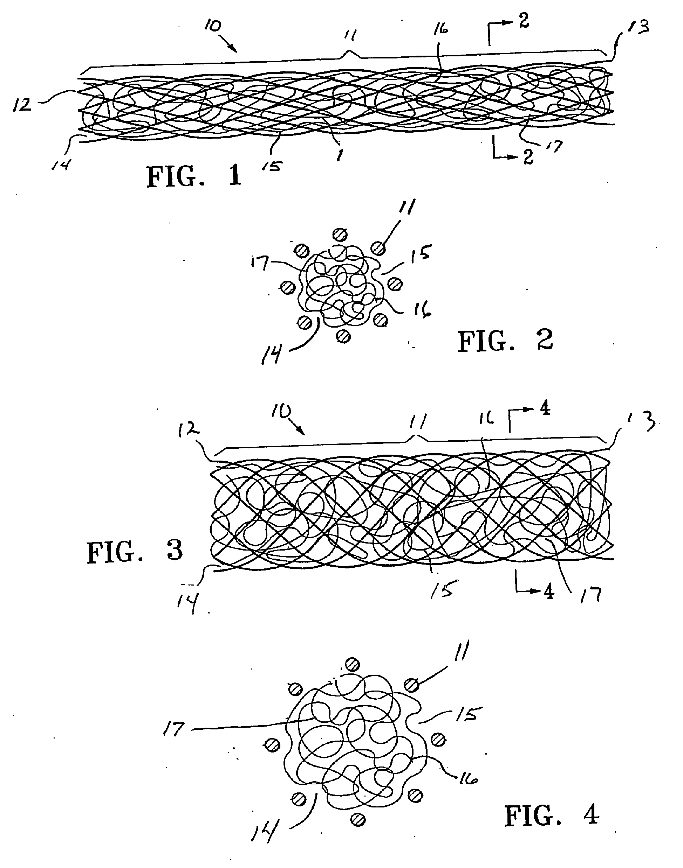

Figures 1-4 schematically illustrate an occluding contraceptive device 10 embodying features of the invention that generally includes an expandable stent-like occluding component or structure 11 having a first open end 12, a second open end 13, a lumen 14 extending therein and a fibrous member 15 within the inner lumen extending along a substantial length thereof. Figures 1 and 2 depict the device 10 in a delivery configuration for introduction into and advancement within the patient's reproductive lumen. Figures 3 and 4 depict the device in an expanded configuration.

As best shown in Figure 2, a fibrous member 15 is transversely disposed in the occluding structure 11. In this embodiment, in order to electrically stimulate tissue growth into or onto the occluding component 11, woven or interspersed wire element 16 is formed of one metallic material and woven or interspersed wire element 17 is formed of a second different metallic material. The metallic wires generate sufficient galvanic activity with respect to the first metallic material to stimulate tissue growth when disposed within a patient's body lumen and in contact with a body or other conductive fluid. For example, wire 16 may be made of stainless steel and wire 17 may be made of copper. While the galvanic activity generated by stainless steel and copper are very slight, the mild electrical conditions generated are sufficient to stimulate tissue ingrowth into or onto the occluding component. As previously mentioned, the copper wires 17 not only generate a galvanic response, but can provide contraceptive effects. To simplify the drawings only the two metallic strands 16 and 17 are shown. The fibrous member 15 may have a plurality of biocompatible, non-metallic strands (e.g. PET, nylon, Hytrel) to facilitate cellular growth to augment that provided by the galvanic activity.

As illustrated in Figure 4, the fibrous member 15 may be configured to expand when the occluding device 10 is expanded to the expanded configuration, so that the fibrous member 15 extends across the expanded lumen 14. The stent-like occluding component 11 has an open, lattice-type structure facilitating tissue ingrowth through the wall thereof in the expanded configuration that facilitates securing the occluding component to the wall defining the reproductive lumen as well as occluding the reproductive lumen. Preferably, the occluding component 11 is of a diameter which is about equal to or slightly larger than the dimensions of the reproductive lumen within which the contraceptive device 10 is to be disposed. For example, for deployment within a female patient's fallopian tubes, the expanded transverse dimensions may be about 0.05 mm to about 5 mm, preferably about 0.1 to about 3 mm.

The fibrous member 15 is permeable to facilitate epithelialization or other tissue ingrowth, and the complex comprising the fibrous member with the tissue ingrowth occludes the reproductive lumen sufficiently to prevent the passage of reproductive cells therethrough.

Alternatively, the metallic strands forming in part the fibrous member 15, e.g. 16 and 17, may be formed of the same metallic material and in contact with the occluding component 11 which is formed of a different metallic material to generate sufficient galvanic activity to stimulate tissue growth.

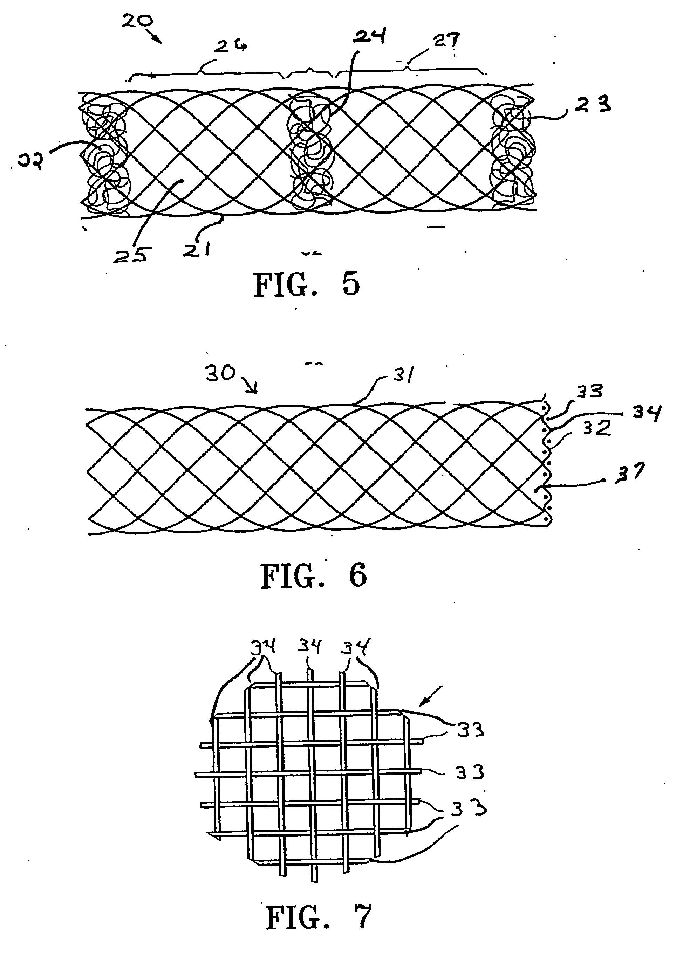

In an alternative embodiment illustrated in FIG. 5, an occluding device 20 has an occluding component 21 (similar to that shown in Figure 1) with a plurality of fibrous members 22, 23 and 24 disposed within the inner lumen 25 of occluding component 21. The individual fibrous members 22-24 are spaced along the length of the tubular occluding component 21 with fibrous member 22 and 23 being within the ends of the occluding component and fibrous member 24 being centrally spaced. Three fibrous members 22-24 are illustrated but a greater or lesser number of fibrous members may be disposed within the inner lumen 25. As discussed above, the individual filaments or strands of a single fibrous member may be formed of different metallic material, the metallic filaments of one fibrous member may be different metallic material from the metallic filaments of an adjacent fibrous member or the metallic filaments of one or more of the fibrous members may be formed of metallic material different from the metallic material of the occluding component 21 in order to develop sufficient galvanic activity to stimulate tissue growth into or onto the device 10.

Additional, the occluding component 21 may have one tubular section 26 formed of a different metallic material than tubular section 27 thereof. In this construction, the filaments of the mesh members may be formed of metallic material different from the metallic material of one or both of the tubular sections 23 and 24 to vary the galvanic activity along the length of the occluding component when disposed in body fluids or other electrically conductive fluids. While not shown, the tubular segments 26 and 27 may be separated and insulated by fibrous members disposed therebetween.

Figures 6 and 7 illustrate an occluding device 30 having an occluding component 31 with a permeable mesh member 32 formed of a plurality of woven strands 33 and 34 which extend across open end 35 of the occluding component 31 and facilitate tissue growth and occlude a body lumen into which the occluding device is deployed. Some or all of the woven strands 33 may be formed of a first metallic material and some or all of woven strands 34 may be formed of a metallic material different from that of strands 33. As discussed above, the metallic strands 33 or 34 of mesh member 32 may be formed of a metallic material different from the metallic material of the occluding component 31.

Figure 7 is an end view of the device illustrated in Figure 6, illustrating the woven strands 33 and 34 forming the mesh member 32. However, the mesh member 32 may comprise a variety of suitable permeable structures which support tissue growth. For example, the mesh member 32 may be replaced with a foamed porous membrane formed of biocompatible materials. The electrical activity stimulates and enhances tissue growth as described above.

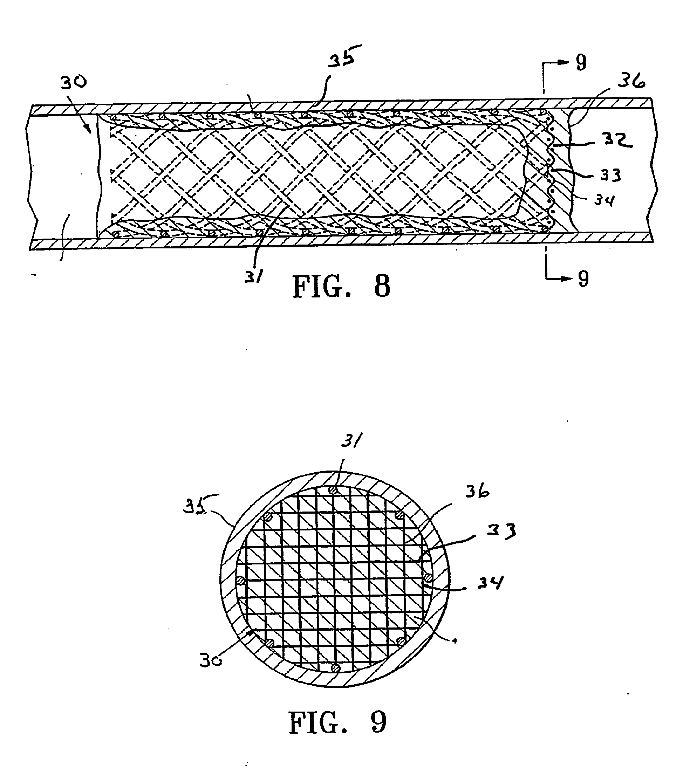

Figures 8 and 9 schematically illustrate the occluding device 30 shown in Figures 6 and 7 within a patient's reproductive lumen 35 in an expanded configuration. Tissue growth 36 is shown within the mesh member 33 disposed at the open end 37 of the occluding component 11. For purposes of simplicity, the embodiment illustrated in Figures 6-9 depicts the mesh member 32 as a single layer of woven filaments or strands 33 and 34, disposed across the opening 37 with one or more wires within the mesh member providing galvanic effect between themselves or in conjunction with the metallic material of the occluding component 31. The mesh member 32 may be formed of a plurality of layers with the individual layers having the same or different mesh sizes. Galvanic action may be generated in the embodiment with the multilayered mesh member between the different layers, within the layers themselves and between the layers and the metallic material of the occluding component. The individual layers of the mesh member may be separated by layers of insulating material. The mesh member 30 is shown as being woven or braided to simplify the drawings. However, the member 30 may be in the form of a fibrous matt or other fibrous or porous construction.

A variety of biocompatible non-metallic materials may be used to form the mesh members 33 and 34 including polymers and treated animal tissues, e.g Dacron, Nylon, heterologous tissue, such as porcine or bovine pericardial tissue, which promote tissue growth, whereas the occluding component is constructed to provide galvanic electrical stimulation to encourage cell proliferation and thus tissue ingrowth by itself or in conjunction with one or more metallic strands in the mesh members 33 and 34. Additionally, the strands 33 and/or 34 of the mesh member 32 may be coated or otherwise impregnated with cell growth stimulators, hormones, and/or chemicals to enhance tissue impregnation. The strands used to form the mesh member 32 are generally about 0.00025 mm to about 0.25 mm in diameter. It would be obvious that a wide variety of mesh sizes that support tissue growth may be used. For example, in one embodiment the mesh member 32 may have a mesh size of about 5 µm to about 0.05 mm, and preferably about 10 µm to about 15 µm. Preferably, mesh members having relatively large mesh sizes are coated with the epithelialization promoter agents.

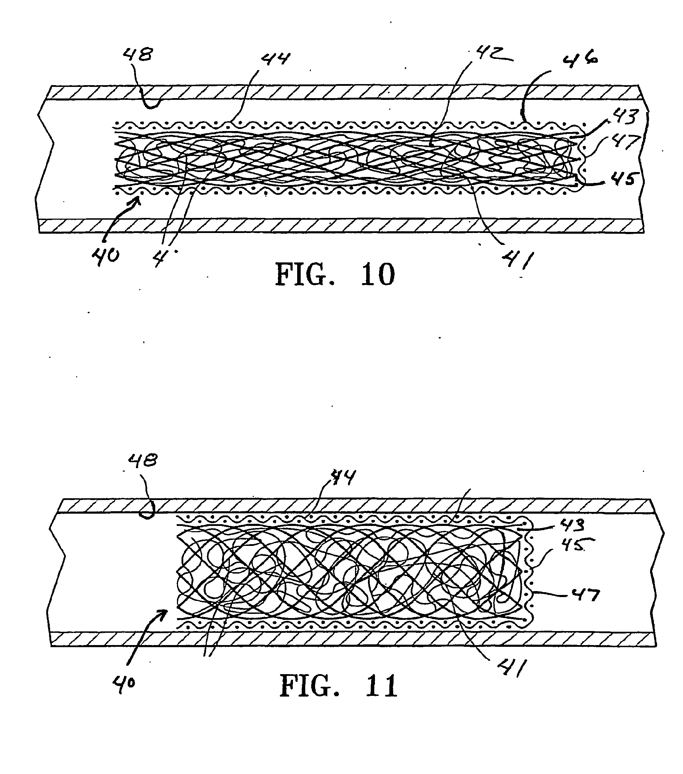

Figures 10 and 11 illustrate an embodiment having features of the invention which has an occluding device 40 (similar to that shown in Figures 6-9) having an occluding component 41 with a fibrous mass 42 provided within the inner lumen 43 of the occluding component and a fibrous jacket or sock 44 that extends along at least a section of the outer surface of the occluding component 41 and over an open end 45 of the occluding component 41. The fibrous material on the exterior, interior and an open end ensures more widespread tissue growth for occlusion. The portion 46 of the fibrous jacket or sock 44 may be formed integral or separate from the portion 47 of the fibrous jacket which extends over the open end 45. Galvanic activity may be generated between metallic strands in the jacket 44, between metallic strands in the jacket 44 and metallic strands within the fibrous body 42 within inner lumen 43 or metallic strands within either the fibrous body 42 or the jacket or sock 44 or both and the occluding component. The fibrous material of the fibrous body 42 and that of the jacket or sock 44 are permeable to allow for tissue ingrowth when the occluding component 41 is expanded into contact with a wall of the reproductive lumen 46 as shown schematically in Figure 11. The occluding component 41 is preferably self expanded within the reproductive lumen 48 due to the presence of the fibrous body 42 within the inner lumen 43.

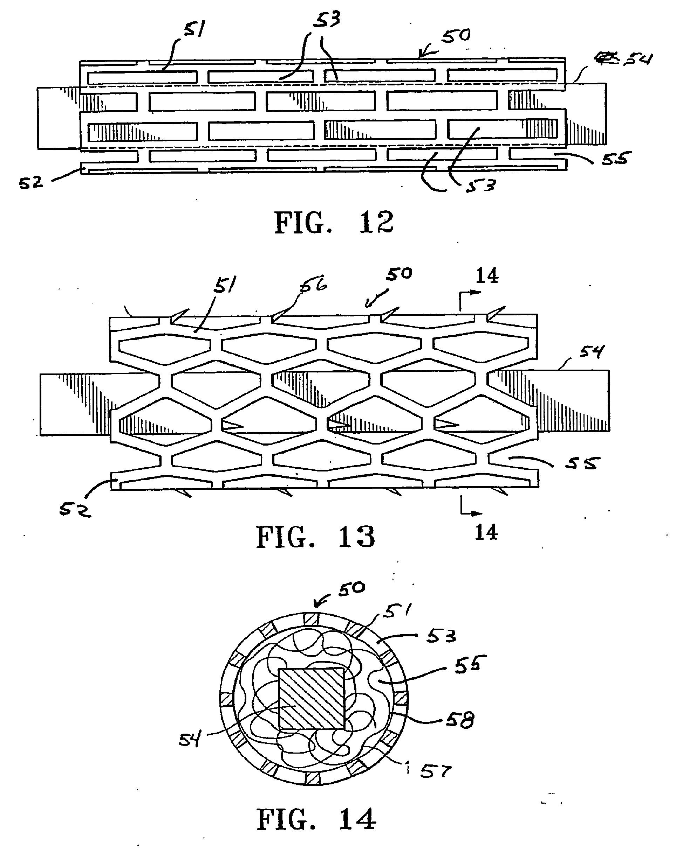

Figures 12-14 illustrate an alternative occluding device 50 embodying features of the invention and having a tubular, stent-like occluding component 51 in the form of a metallic hypotube 52 with slots 53 cut into the wall of the hypotube to allow expansion of the occluding component to an expanded configuration with larger transverse dimensions as shown in Figure 13. In this construction, a rod or wire 54 is longitudinally disposed within the inner lumen 55 of the occluding component 51. The galvanic activity may be created by constructing all or part of the occluding component 51 of one metal and the rod or wire 54 disposed within the inner lumen 55 of the occluding component formed of a different metal. The occluding component 51 may optionally have barbed or hook like projections 56 that aid in retention of the occluding component 51 within a reproductive lumen. While the projections 56 are shown in Figure 13 inclined toward one end of the occluding component, they may be inclined toward either or both ends of the occluding component. The metal rod or wire 54 may be suspended within a fibrous body 57 as shown in Figure 14. The fibrous body 57 may be formed of metallic or non-metallic strands 58, e.g. polyethylene terephthalate (PET). These fibrous strands 58 may be formed of insulating material to isolate the rod or wire 54 from the occluding component 51. However, the fibrous body 57 may have one or more metallic strands which are formed of different metallic material from either the rod or wire 54 or the occluding component 51.

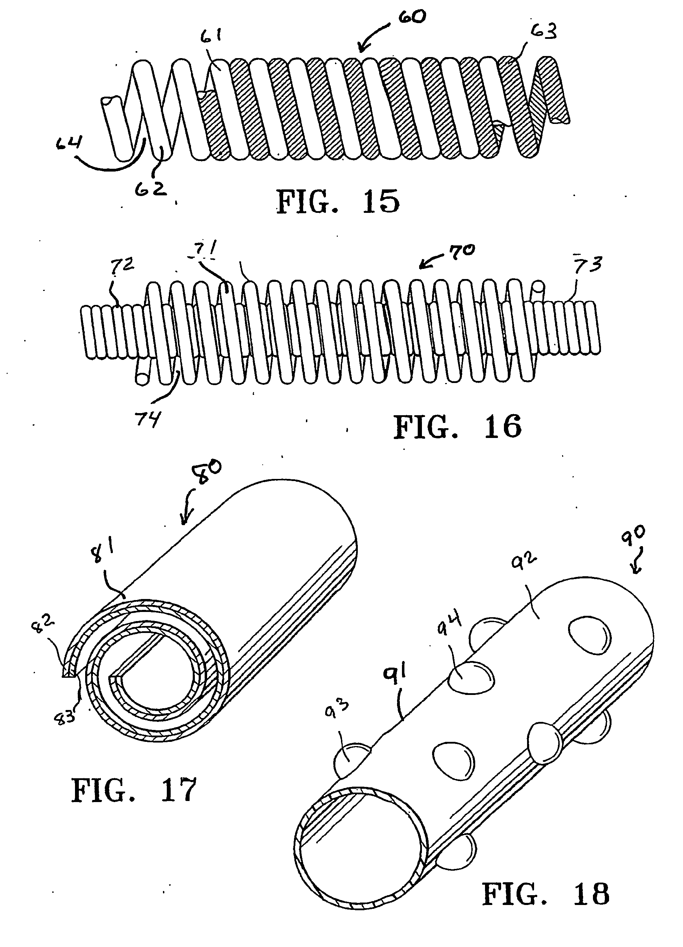

Figure 15 illustrates an alternative occluding device 60 having features of the invention. In this embodiment the occluding device 60 comprises an occluding component 61 formed into a helically shaped coil 62 of a first metallic material with space 63 between individual turns of the helically shaped coil. A second helically shaped coil 64 formed of a second metallic material different from the first metallic material is fitted into the space 63 of the first coil 62 to generate galvanic activity to enhance tissue growth into and/or on the occluding component 61. The contacting surfaces of the helically shaped coils 62 and 64 may be insulated (not shown) to varying degrees to control the level of galvanic activity to achieve the desired tissue ingrowth. As previously described, a fibrous body (not shown) may be disposed within the inner lumen 65 of the occluding component 61 or a fibrous jacket or sock (such as shown in Figures 10 and 11) may be provided on the exterior of the occluding component to further enhance tissue growth. The helical coils 62 and 64 may be balloon or self expandable for deployment within a patient's body lumen.

Another occluding device 70 embodying features of the invention is depicted in Figure 16, which has an occluding component 71 formed of a helically shaped coil structure 72 similar to the occluding component 61 shown in Figure 15 and a smaller diameter helical coil 73 disposed within the inner lumen 74 of the occluding component 71. The occluding component 71 is expandable and preferably is formed of shape memory or superelastic metallic material such as NiTi alloy like NITINOL. The helically shaped coil 72 may be suspended within the inner lumen 73 by a mesh or other fibrous body (not shown) such as discussed above and shown in Figure 14. The inner coil 72 may be formed of metallic material different from the metallic material of the occluding component to generate galvanic activity which stimulates tissue growth into and onto the occluding component 71 of device 70. The strands of a fibrous body disposed within the inner lumen 73 may be metallic strands and add to the electrical activity.

Another occluding device 80 embodying features of the invention is shown in Figure 17. In this embodiment, the occluding device 80 has an occluding component 81 having two layers 82 and 83 formed of different metals rolled together. There may be a layer of insulative material between the two metal layers (not shown) to enhance the galvanic effects and to localize the ionic exchange to the edges of the occluding component 81.

Yet another construction embodying features of the invention is shown in Figure 18. The occluding device 90 of this embodiment has an occluding component 91 which is a tubular element 92 that may or may not be expandable against the walls of the body lumen with particles 93 and 94 formed of different metallic material on the surface of the tubular member. The tubular element 92 is generally an insulator, and when the device is implanted in a reproductive lumen and in contact with body fluids which act as electrolytes, a voltage differential exists between the different metal particles which is sufficient to encourage tissue ingrowth to occur, and to thus enhance the occlusion of the reproductive lumen into which the occluding device 90 is deployed.

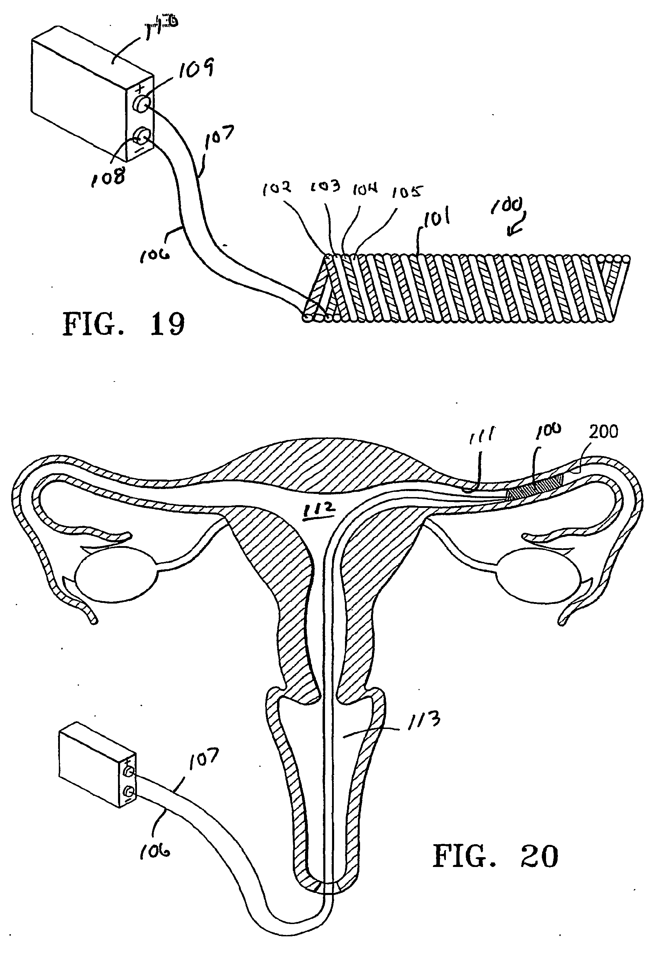

In addition to the stimulation of tissue growth by galvanic activity as discussed above, a voltage may be impressed against the tissue of a reproductive lumen such as a fallopian tube or vas deferens to enhance tissue growth into or onto an occluding device deployed within the reproductive lumen such as described herein. A suitable occluding device 100 is shown in Figures 19 and 20. As shown in Figure 19 the suitable occluding device 100 comprises an occluding component 101 formed of four separate interfitting helical coils 102, 103, 104 and 105. Helical coils 102 and 104 are electrically connected by conductors 106 and 107 respectively to opposite poles 108 and 109 of a power source 110 (e.g. battery). The helical coils 102 and 104 act as electrodes. Helical coils 103 and 105 are insulators. In this way the two helical coils 102 and 104 are insulated from each other along their length, and conduct electrical current to each other through the tissue in contact with metallic portions of the occluding component 101.

Figure 20 illustrates the occluding device 100 deployed within a patient's fallopian tube 111. The conductors 106 and 107 extend through the patient's uterine cavity 112 and vaginal canal 113 and are electrically connected to battery 110. The application of electrical power from the external source 110 is usually intermittent. For example, electrical power from the battery 110 can be applied for about one hour a day for the first five days and may only be needed to stimulate initial tissue ingrowth. This may be monitored and the application of the electrical power discontinued when the tissue ingrowth is sufficiently complete. This electrical stimulation is very helpful in initiating effective tissue ingrowth at the inception of the implantation of the occlusive device. Essentially the same basic system may be employed to occlude a male reproductive lumen such as a vas deferens.

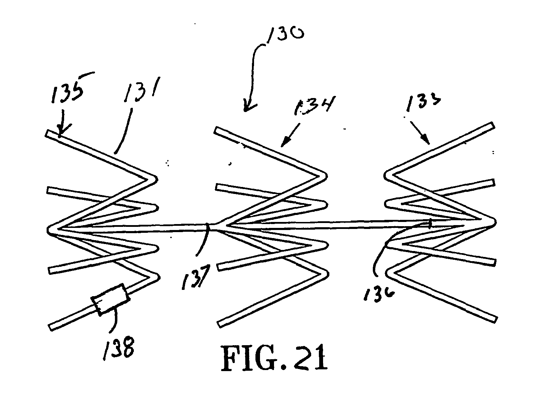

Another occluding device 130 is shown in Figure 21 which has features of the invention. The occluding device 130 has an occluding component 131 with an elongated shaft 132 and spider-like expandable elements 133, 134 and 135. The expandable elements are formed of different metallic materials than the expandable element adjacent thereto to provide the desired galvanic activity. The junctions between the portions of differing metallic materials are shown at locations 136 and 137. The junctions 136 and 137 may be formed mechanically, metallurgically, adhesively, by laser welding. In those situations in which the portions of the shaft are difficult to join, a cylindrical collar may be employed to joint the mating ends of the shaft sections. Alternatively, the occluding component 131 may be formed entirely of a first metallic material and a collar 138 formed of a second different metallic material may be secured to one or more of the expandable legs 139 of expandable spider-like element 135 or the shaft 132 to provide the galvanic activity required to enhance tissue growth. One or more of the spider-like elements 133-135 may have a fibrous member (not shown) such as shown in Figure 5.

Various means may be employed to secure an occluding device embodying features of the invention within a reproductive or other lumen other than as described above. For example, mechanical, adhesive or other anchoring means may be employed to secure the expanded occluding device to the vessel wall defining the body lumen. Means to secure a stent or prosthetic device to an aortic or arterial wall, such as described in U.S. Pat. No. 4,140,126; U.S. Pat. No. 4,562,596; U.S. Pat. No. 4,577,631; U.S. Pat. No. 4,787,899; U.S. Pat. No. 5,104,399; U.S. Pat. No. 5,167,614; U.S. Pat. No. 5,275,622; U.S. Pat. No. 5,456,713; and U.S. Pat. No. 5,489,295 may be useful in this regard.

The occluding component of devices embodying features of the invention are preferably formed of a superelastic material such as NiTi alloy so as to provide a controlled force on the body lumen during expansion. Additionally, the surface of the occluding components may be configured to further facilitate epithelial and other tissue ingrowth. Suitable surface treatments include plasma etching, sand blasting, machining and other treatments to roughen the surface. The surface of the occluding component may be coated or seeded to spur epithelialization.

The fibrous and mesh members may be connected to the occluding component by a variety of suitable means including tying, sutures, clips, adhesives, heat bonding, or solvent bonding.

Various modifications and improvements may be made to the present invention without departing from the scope thereof. For example, while the invention has been discussed primarily in terms of occluding a reproductive body lumen, the occluding device may be used to occlude a variety of body lumens or passageways such as arteries or veins in a variety of situations, the nidus of an arterial-venous malformation, patent ductus arteriosis in infants, as well as arteries leading to benign or cancerous tumors.

Additionally, other occluding devices and delivery devices that may be utilized are disclosed in Patent Applications Serial Number 08/770,123, filed on December 18, 1996, Serial No. 09/112,085, filed on July 8, 1998, Serial No. 09/468,749, filed on December 21, 1999, Serial No. 10/746,131, filed on December 24, 2004 and Provisional Application Serial No. 60/483,587, filed on June 27, 2003.

Although individual features of the invention may be described with respect to one or more of the embodiments but not in other embodiments, it should be readily apparent that individual features of one embodiment having features of the invention can be combined with any or all the features of one or more of other embodiments.

## Claims

1. A contraceptive device for occluding a patient's reproductive lumen, comprising:
a. an expandable occluding component (11, 21, 31, 41, 51, 61, 71, 81, 91, 131);
b. a first element (16, 33, 62, 71) which is associated with the occlusion component and which is formed at least in part of a first metallic material; and
c. a second element (17, 34, 64, 72) which is associated with the occlusion component;
**characterized in that**:
the second element (17, 34, 64, 72) is formed at least in part of a second metallic material different from the first metallic material; and
d. an electrical connection between the first and second metallic elements configured to generate sufficient galvanic activity between the first and second metallic elements to stimulate tissue growth within the reproductive lumen when in contact with conductive fluid within the reproductive lumen.

2. The device of Claim 1 wherein:
(a) the occluding component (11, 21, 31, 41, 51) is a stent; and preferably wherein at least one of the first or second elements (44) are formed integral with the stent;
(b) one of the first and second element is formed at least in part of iron or copper;
(c) said first element is contained within said second element; or
(d) at least one of the elements (62, 64, 71, 72) is helically shaped.

3. The device of Claim 1 wherein both of the first and second elements are helically shaped; and preferably
(a) wherein one helically shaped element (72) is contained within another helically shaped element (71); or
(b) wherein one helically shaped element (64) is disposed within coils of another helically shaped element (62) along at lease a part of the length of the device.

4. The device of Claim 1 wherein the occluding component (11, 21, 31, 41, 51) is provided with fibrous member (15, 22, 23, 24, 32, 42, 44, 57) having a plurality of strands (16, 17, 33,34) secured thereto.

5. The device of Claim 4 wherein:
(a) one of the strands (16, 33) of the fibrous member is the first metallic element, preferably wherein one of the strands (17, 34) of the fibrous member is the second metallic element;
(b) the fibrous member has a plurality of strands in a mesh construction (32);
(c) the fibrous member is disposed within an inner lumen (14, 25, 43) of the occluding component; or
(d) the fibrous member (44) is disposed on the occluding component.

6. The device of Claim 1 wherein;
(a) the first metallic element is a granule (93, 94);
(b) both the first and second metallic elements are granules (93, 94);
(c) the occluding component (91) is a tubular member (92) and the first and second elements are granules (93, 94) disposed on an exterior surface of the tubular member, or
(d) the first element (82) is a planar member, the second element (83) is a planar member and the first and second elements (82, 83) are coplanar along at least a portion of each other; preferably wherein said first and second elements are secured together along at least part of the co-planar portions thereof.

7. The device of Claim 1 wherein the occluding component (131) has elongated shaft (132) and a plurality of expandable spider like elements (131, 133, 134) are provided along the elongated shaft.

8. The device of Claim 7 wherein one section of the occluding component (131) having one spider-like element is formed at least in part of a first metallic material and a second section of the occluding component is formed of a second metallic material different from the first metallic material,

9. The device of Claim 7 wherein the occluding component (131) is formed of a first metallic material and an element (138) formed of a second metallic material different from the first metallic material is secured to the occluding component; preferably wherein the element (138) formed of a second metallic material is in the form of a collar or sleeve secured to the occluding component.

10. A contraceptive device for occluding a patient's reproductive lumen, comprising:
a. an expandable occluding component;
b. a first metallic element (102) which is associated with the occluding component; and
c. a second metallic element (104) which is associated with the occluding component;
**characterized in that** the device comprises:
d. an electrical power source (110);
e. a first electrical conductor (106) electrically connected between the first metallic element and the power source; and
f. a second electrical conductor (107) electrically connected between the second metallic element and the electrical power source, the metallic elements being insulated from each other so that a voltage may be impressed against the tissue of the reproductive lumen such as a fallopian tube or vas deferens to enhance tissue growth into or onto an accluding device deployed within the reproductive lumen.

11. The device of Claim 10 wherein the electrical power source is a battery.

12. The device of Claim 10 wherein the first and second metallic elements are in the form of first and second helical coils respectively; preferably wherein the first helical coil (102) has space between adjacent turns of the helical coil; and preferably wherein the second helical coil (107) is fitted within the space between the turns of the first helical coil.

## Patentansprüche

1. Empfängnisverhütende Vorrichtung zum Verschließen des Fortpflanzungslumens eines Patienten, mit:
a. einer dehnbaren Verschließkomponente (11, 21, 31, 41, 51, 61, 71, 81, 91, 131);
b. einem der Verschließkomponente zugeordneten ersten Element (16, 32, 62, 71), welches zumindest teilweise aus einem ersten metallischen Material gebildet ist; und
c. einem der Verschließkomponente zugeordneten zweiten Element (17, 34, 64, 72);
**dadurch gekennzeichnet, dass**:
das zweite Element (17, 34, 64, 72) zumindest teilweise aus einem zweiten metallischen Material gebildet ist, welches vom ersten metallischen Material verschieden ist; und
d. mit einer elektrischen Verbindung zwischen dem ersten und dem zweiten metallischen Element, die konfiguriert ist, um eine ausreichende galvanische Aktivität zwischen dem ersten und dem zweiten metallischen Element zu erzeugen, um bei Kontakt mit leitfähigem Fluid innerhalb des Fortpflanzungslumens das Gewebewachstum innerhalb des Fortpflanzungslumens zu stimulieren.

2. Vorrichtung nach Anspruch 1, wobei:
(a) die Verschließkomponente (11, 21, 31, 41, 51) ein Stent ist; und wobei vorzugsweise zumindest eines der beiden Elemente das erste und/oder das zweite Element (44), integral mit dem Stent ausgebildet ist;
(b) eines der zwei Elemente zumindest teilweise aus Eisen oder Kupfer gebildet ist;
(c) das erste Element im zweiten Element enthalten ist; oder
(d) zumindest eines der Elemente (62, 64, 71, 72) schraubenlinienförmig ausgebildet ist.

3. Vorrichtung nach Anspruch 1, wobei sowohl das erste als auch das zweite Element schraubenlinienförmig ausgebildet sind; und wobei vorzugsweise
(a) ein schraubenlinienförmig ausgebildetes Element (72) in einem anderen schraubenlinienförmig ausgebildeten Element (71) enthalten ist;
oder
(b) ein schraubenlinienförmig ausgebildetes Element (64) entlang zumindest eines Teiles der Länge der Vorrichtung innerhalb der Windungen eines anderen schraubenlinienförmig ausgebildeten Elements (62) angeordnet ist.

4. Vorrichtung nach Anspruch 1, wobei die Verschließkomponente (11, 21, 31, 41, 51) mit Faserelementen (15, 22, 23, 24, 32, 42, 44, 57) versehen ist, die eine Mehrzahl von daran befestigten Strängen (16, 17, 33, 34) aufweisen.

5. Vorrichtung nach Anspruch 4, wobei:
(a) einer der Stränge (16, 33) des Faserelements das erste metallische Element ist, wobei vorzugsweise einer der Stränge (17, 34) des Faserelements das zweite metallische Element ist;
(b) das Faserelement eine Mehrzahl von Strängen in einer Maschenstruktur (32) aufweist;
(c) das Faserelement innerhalb eines Innenlumens (14, 25, 43) der Verschließkomponente angeordnet ist; oder
(d) das Faserelement (44) auf der Verschließkomponente angeordnet ist.

6. Vorrichtung nach Anspruch 1, wobei:
(a) das erste metallische Element ein Körnchen (93, 94) ist;
(b) sowohl das erste als auch das zweite metallische Element Körnchen (93, 94) sind;
(c) die Verschließkomponente (91) ein rohrförmiges Element (92) ist und das erste und das zweite Element Körnchen (93, 94) sind, die auf einer Außenfläche des rohrförmigen Elements angeordnet sind; oder
(d) das erste Element (82) ein planares Element ist, das zweite Element (83) ein planares Element ist und das erste und das zweite Element (82, 83) jeweils entlang zumindest eines Teiles von ihnen koplanar sind; wobei vorzugsweise das erste und das zweite Element entlang zumindest eines Teiles ihrer koplanaren Teile aneinander befestigt sind.

7. Vorrichtung nach Anspruch 1, wobei die Verschließkomponente (131) einen länglichen Schaft (132) aufweist und eine Mehrzahl von dehnbaren spinnenartigen Elementen (131, 133, 134) entlang dem länglichen Schaft vorgesehen sind.

8. Vorrichtung nach Anspruch 7, wobei ein Abschnitt der Verschließkomponente (131) mit einem spinnenartigen Element zumindest teilweise aus einem ersten metallischen Material gebildet ist und ein zweiter Abschnitt der Verschließkomponente aus einem zweiten metallischen Material gebildet ist, welches vom ersten metallischen Material verschieden ist.

9. Vorrichtung nach Anspruch 7, wobei die Verschließkomponente (131) aus einem ersten metallischen Material gebildet ist und ein Element (138), welches aus einem zweiten, vom ersten metallischen Material unterschiedlichen metallischen Material gebildet ist, an der Verschließkomponente befestigt ist; wobei vorzugsweise das aus einem zweiten metallischen Material gebildete Element (138) in Form einer Manschette oder Hülse vorliegt, die an der Verschließkomponente befestigt ist.

10. Empfängnisverhütende Vorrichtung zum Verschließen des Fortpflanzungslumens eines Patienten, mit:
a. einer dehnbaren Verschließkomponente ;
b. einem der Verschließkomponente zugeordneten ersten metallischen Element (102);
c. einem der Verschließkomponente zugeordneten zweiten metallischen Element (104);
**dadurch gekennzeichnet, dass** die Vorrichtung aufweist:
d. eine Stromquelle (110);
e. einen ersten elektrischen Leiter (106), der elektrisch zwischen dem ersten metallischen Element und der Stromquelle angeschlossen ist; und
f. einen zweiten elektrischen Leiter (107), der elektrisch zwischen dem zweiten metallischen Element und der Stromquelle angeschlossen ist, wobei die metallischen Elemente voneinander isoliert sind, so dass eine Spannung auf das Gewebe des Fortpflanzungslumens wie des Eileiters oder des Samenleiters aufgeprägt werden kann, um das Gewebewachstum in oder auf eine in dem Fortpflanzungslumen eingesetzte Verschließeinrichtung zu fördern.

11. Vorrichtung nach Anspruch 10, wobei die Stromquelle eine Batterie ist.

12. Vorrichtung nach Anspruch 10, wobei das erste und das zweite metallische Element in Form von ersten bzw. zweiten schraubenlinienförmigen Wicklungen vorliegen; wobei die erste schraubenlinienförmige Wicklung (102) vorzugsweise einen Raum zwischen benachbarten Windungen der schraubenlinienförmigen Wicklung aufweist; und wobei die zweite schraubenlinienförmige Wicklung (104) vorzugsweise in dem Raum zwischen den Windungen der ersten schraubenlinienförmigen Wicklung eingefügt ist.

## Revendications

1. Dispositif contraceptif pour obstruer la lumière reproductrice d'un patient, comprenant :
a. un composant d'occlusion dilatable (11, 21, 31, 41, 51, 61, 71, 81, 91, 131) ;
b. un premier élément (16, 33, 62, 71) qui est associé au composant d'occlusion et qui est formé au moins en partie d'un premier matériau métallique; et
c. un second élément (17, 34, 64, 72) qui est associé au composant d'occlusion;
**caractérisé en ce que**
le second élément (17, 34, 64, 72) est formé au moins en partie d'un second matériau métallique différent du premier matériau métallique ; et
d. une connexion électrique entre le premier et le second éléments métalliques, configurés de façon à générer une activité galvanique suffisante entre le premier et le second éléments métalliques, afin de stimuler la croissance des tissus dans la lumière reproductrice, en cas de contact avec un fluide conducteur dans la lumière reproductrice.

2. Dispositif selon la revendication 1, dans lequel :
(a) le composant d'occlusion (11, 21, 31, 41, 51) est un stent ; et de préférence dans lequel au moins un des premier et second éléments (44) est formé d'un seul tenant avec le stent ;
(b) un des premier et second éléments est formé au moins en partie de fer ou de cuivre ;
(c) ledit premier élément est contenu dans ledit second élément ; ou
(d) au moins un des éléments (62, 64, 71, 72) est de forme hélicoïdale.

3. Dispositif selon la revendication 1, dans lequel le premier et le second éléments sont de forme hélicoïdale et de préférence
(a) dans lequel un élément de forme hélicoïdale (72) est contenu dans un autre élément de forme hélicoïdale (71) ou
(b) dans lequel un élément de forme hélicoïdale (64) est disposé dans des bobines d'un autre élément de forme hélicoïdale (62) le long au moins d'une partie de la longueur du dispositif.

4. Dispositif selon la revendication 1, dans lequel le composant d'occlusion (11, 21, 31, 41, 51) est doté d'un élément fibreux (15, 22, 23, 24, 32, 42, 44, 57) ayant une pluralité de brins (16, 17, 33, 34) fixé à celui-ci.

5. Dispositif selon la revendication 4, dans lequel :
(a) un des brins (16, 33) de l'élément fibreux est le premier élément métallique, de préférence dans lequel un des brins (17, 34) de l'élément fibreux est le second élément métallique;
(b) l'élément fibreux a une pluralité de brins dans une construction en maille (32) ;
(c) l'élément fibreux est disposé dans une lumière interne (14, 25, 43) du composant d'occlusion; ou
(d) l'élément fibreux (44) est disposé sur le composant d'occlusion.

6. Dispositif selon la revendication 1, dans lequel:
(a) le premier élément métallique est un granule (93, 94) ;
(b) le premier et le second éléments métalliques sont des granules (93,94) ;
(c) le composant d'occlusion (91) est un élément tubulaire (92) et le premier et le second éléments sont des granules (93, 94) disposés sur une surface extérieure de l'élément tubulaire, ou
(d) le premier élément (82) est un élément plan, le second élément (83) est un élément plan et le premier et le seconds éléments (82, 83) sont coplanaires, le long au moins d'une partie l'un de l'autre ; de préférence dans lequel ledit premier et ledit second éléments sont fixés ensemble le long au moins d'une partie des parties coplanaires correspondantes.

7. Dispositif selon la revendication 1, dans lequel le composant d'occlusion (131) dispose d'un arbre allongé (132) et une pluralité d'éléments semblables à des araignées extensibles (131, 133, 134) sont ménagés le long de l'arbre allongé.

8. Dispositif selon la revendication 7, dans lequel une section du composant d'occlusion (131) ayant un élément semblable à une araignée est formé au moins en partie d'un premier matériau métallique et une seconde section du composant d'occlusion est formée d'un second matériau métallique, différent du premier matériau métallique.

9. Dispositif selon la revendication 7, dans lequel le composant d'occlusion (131) est formé d'un premier matériau métallique et un élément (138) formé d'un second matériau métallique différent du premier matériau métallique est fixé au composant d'occlusion ; de préférence dans lequel l'élément (138) formé d'un second matériau métallique est en forme de collier ou de manchon fixé au composant d'occlusion.

10. Dispositif contraceptif visant à obstruer une lumière reproductrice d'un patient, comprenant:
a. un composant d'occlusion dilatable ;
b. un premier élément métallique (102) qui est associé au composant d'occlusion ; et
c. un second élément métallique (104) qui est associé au composant d'occlusion ;
**caractérisé en ce que** le dispositif comprend :
d. une source d'alimentation électrique (110) ;
e. un premier conducteur électrique (106) électriquement raccordé entre le premier élément métallique et la source d'alimentation; et
f. un second conducteur électrique (107) électriquement raccordé entre le second élément métallique et la source d'alimentation électrique, les éléments métalliques étant isolés l'un de l'autre, de sorte qu'une tension puisse être imprimée contre le tissus de la lumière reproductrice telle qu'une Trompe de Fallope ou un canal déférent, afin d'augmenter la croissance tissulaire dans ou sur un dispositif d'occlusion déployé dans la lumière reproductrice.

11. Dispositif selon la revendication 10 dans lequel la source d'alimentation électrique est une batterie.

12. Dispositif selon la revendication 10 dans lequel le premier et le second éléments métalliques sont en forme d'une première et seconde bobines hélicoïdales respectivement ; de préférence dans lequel la première bobine hélicoïdale (102) a un espace entre des tours adjacents de la bobine hélicoïdale ; et de préférence dans lequel la seconde bobine hélicoïdale (107) est installée dans l'espace entre les tours de la première bobine hélicoïdale.
